(19) Europäisches Patentamt
European Patent Office
Office européen des brevets

(11) Veröffentlichungsnummer: **0 469 374 A2**

(12) # EUROPÄISCHE PATENTANMELDUNG

(21) Anmeldenummer: **91111810.7**

(51) Int. Cl.5: **C07D 205/04**, A01N 43/44

(22) Anmeldetag: **16.07.91**

(30) Priorität: **30.07.90 DE 4024118**

(43) Veröffentlichungstag der Anmeldung:
**05.02.92 Patentblatt 92/06**

(84) Benannte Vertragsstaaten:
**BE CH DE DK FR GB IT LI NL**

(71) Anmelder: **BASF Aktiengesellschaft**
**Carl-Bosch-Strasse 38**
**W-6700 Ludwigshafen(DE)**

(72) Erfinder: **Rentzea, Costin, Dr.**
**Richard-Kuhn-Strasse 1-3**
**W-6900 Heidelberg(DE)**
Erfinder: **Rademacher, Wilhelm, Dr.**
**Austrasse 1**
**W-6703 Limburgerhof(DE)**

(54) **Oxalsäurederivate, ihre Herstellung und sie enthaltende Mittel zur Regulierung des Pflanzenwachstums.**

(57) Oxalsäurederivate der Formel I,

A-CO-COO-Y-X-D I

in der die Substituenten folgende Bedeutung haben:

A ein ggf. subst. über sein N-Atom gebundener Azetidinring;
Y ggf. subst. Alkylen;
X Sauerstoff oder Schwefel;
D ggf. subst. Alkyl oder Alkenyl;
monocyclisches oder polycyclisches ggf. subst Cycloalkyl, Cycloalkylmethyl, Cycloalkenyl oder Cycloalkenylmethyl;
ggf. subst. Phenyl, Phenyl-alkyl oder Phenyl-alkenyl,

ihre Herstellung und sie enthaltende wachstumsregulierende Mittel.

EP 0 469 374 A2

Die vorliegende Erfindung betrifft Oxalsäurederivate der allgemeinen Formel I

A-CO-COO-Y-X-D I,

in der die Substituenten folgende Bedeutung haben:

- A ein über sein N-Atom gebundener Azetidinring, welcher ein oder zwei $C_1$-$C_3$-Alkylgruppen tragen kann;
- Y $C_2$-$C_{10}$-Alkylen, welches ein bis drei $C_1$-$C_3$-Alkylgruppen tragen kann;
- X Sauerstoff oder Schwefel;
- D $C_1$-$C_{20}$-Alkyl oder $C_3$-$C_{10}$-Alkenyl, wobei diese Gruppen ein bis fünf Halogenatome tragen können; monocyclisches oder polycyclisches $C_3$-$C_{10}$-Cycloalkyl, $C_3$-$C_{10}$-Cycloalkylmethyl, $C_5$-$C_{10}$-Cycloalkenyl oder $C_5$-$C_{10}$-Cycloalkenylmethyl, wobei diese Ringe ein bis drei $C_1$-$C_4$-Alkylgruppen oder einen Phenylring tragen können;
  Phenyl, Phenyl-$C_1$-$C_6$-alkyl oder Phenyl-$C_3$-$C_6$-alkenyl, wobei die aromatischen Reste ein bis fünf Halogenatome und/oder ein bis drei der folgenden Gruppen tragen können: Nitro, $C_1$-$C_4$-Aklyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio.

Außerdem betrifft die Erfindung Verfahren zur Herstellung dieser Oxalsäurederivate, sie enthaltende Mittel und Verfahren zu ihrer Verwendung als Pflanzenwachstumsregulatoren.

Oxalsäurederivate sind aus der Literatur als Synthesezwischenprodukte bekannt.

Aufgabe der vorliegenden Erfindung waren neue wirksame Pflanzenwachstumsregulatoren.

Demgemäß wurden die eingangs definierten Oxalsäurederivate I gefunden. Außerdem wurden Verfahren zur Herstellung dieser Oxalsäurederivate, sie enthaltende Mittel und Verfahren zu ihrer Verwendung als Pflanzenwachstumsregulatorengefunden.

Die Oxalsäurederivate I sind auf verschiedenen Wegen zugänglich. Besonders vorteilhaft erhält man sie nach einem der im nachfolgenden beschriebenen Verfahren A bis D.

Verfahren A:

Man erhält Verbindungen der Formel I beispielsweise dadurch, daß man ein Oxalylesterhalogenid der allgemeinen Formel II in an sich bekannter Weise (Caro et al., Chem. Ber., 63, 1534 (1930)) in einem inerten organischen Lösungsmittel in Gegenwart eine Base mit einem Azetidinderivat der allgemeinen Formel III umsetzt.

$$\underset{II}{\text{Hal-CO-COO-Y-X-D}} + \underset{III}{\text{A-H}} \longrightarrow \underset{I}{\text{A-CO-COO-Y-X-D}}$$

Hal in der Formel II steht dabei für ein Halogenatom wie Fluor, Chlor, Brom und Jod, vorzugsweise für Chlor oder Brom.

Die Umsetzung erfolgt im allgemeinen bei Temperaturen von -10 bis 80 °C, vorzugsweise bei 0 bis 50 °C.

Als Lösungsmittel eignen sich beispielsweise Halogenkohlenwasserstoffe, insbesondere Chlorkohlenwasserstoffe, z.B. 1,1,2,2,-Tetrachlorethylen oder 1,1,2,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, m-, p-Dichlorbenzol, o-, m-, p-Dibrombenzol, o-, m-, p-Chlortoluol, 1,2,4-Trichlorbenzol; Ether, z.B. Ethylpropylether, Methyl-tert.-butylether, n-Butylethylether, Di-n-butylether, Di-isobutylether, Diisoamylether, Diisopropylether, Anisol, Phenethol, Cyclohexylmethylether, Diethylether, Ethylenglykoldimethylether, Tetrahydrofuran, Dioxan, Thioanisol; Nitrokohlenwasserstoffe wie Nitromethan, Nitroethan, Nitrobenzol, o-, m-, p-Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, z.B. Heptan, Pinan, Nonan, o-, m-, p-Cymol, Benzinfraktionen innerhalb eines Siedepunktintervalles von 70 bis 190 °C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; Ester z.B. Ethylacetat, Acetessigester, Isobutylacetat; Amide, z.B. Formamid, Methylformamid, Dimethylformamid; Ketone, z.B. Aceton, Methylethylketon, gegebenenfalls auch Wasser

und entsprechende Gemische in Betracht. Zweckmäßig verwendet man das Lösungsmittel in einer Menge von 100 bis 2000 Gew.-%, vorzugsweise von 200 bis 700 Gew.-%, bezogen auf Ausgangsstoff III.

Als Basen kommen beispielsweise Kaliumhydroxid, Natriumhydroxid, Kaliumcarbonat, Natriumcarbonat, Lithiumhydroxid, Lithiumcarbonat, Natriumbicarbonat, Kaliumbicarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumbicarbonat, Magnesiumacetat, Zinkhydroxid, Zinkoxid, Zinkcarbonat, Zinkbicarbonat, Zinkacetat, Natriumformiat, Natriumacetat, Trimethylamin, Triethylamin, Tripropylamin, Triisopropylamin, Tributylamin, Triisobutylamin, Tri-sec.-butylamin, Tri-tert.-butylamin, Tribenzylamin, Tricyclohexylamin, Triamylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-4-aminopyridin, N,N-Diethyl-4-aminopyridin, N,N-Dipropyl-4-aminopyridin, N-Methylpyrrolidin, N-Ethylpyrrolidin, N-Methylpiperidin, N-Ethylpiperidin, N-Methyl-pyrrolidon, N-Ethylpyrrolidon, N-Methylimidazol, N-Ethylimidazol, N-Methylmorpholin, N-Ethylmorpholin, N-Methylhexamethylenimin, N-Ethylhexamethylenimin, Pyridin, Chinolin, alpha-Picolin, β-Picolin, gamma-Picolin, Isochinolin, Pyrimidin, Acridin, N,N,N',N'-Tetramethylethylendiamin, N,N,N',N'-Tetraethylethylendiamin, Chinoxalin, Chinazolin, N-Propyldiisopropylamin, N,N-Dimethylcyclohexylamin, 2,6-Lutidin, 2,4-Lutidin, Triethylendiamin. Zweckmäßig verwendet man den Säureakzeptor in stöchiometrischen Mengen, im Überschuß oder Unterschuß von jeweils bis zu 20 mol.-%, bezogen auf den Ausgangsstoff II.

Verfahren B:

Man erhält die Verbindungen der Formel I beispielsweise auch dadurch, daß man ein Oxalylamidhalogenid der allgemeinen Formel IV in an sich bekannter Weise in einem inerten organischen Lösungsmittel in Gegenwart eine Base mit einer Verbindung der allgemeinen Formel V umsetzt.

$$\underset{IV}{A\text{-}CO\text{-}CO\text{-}Hal} \quad + \quad \underset{V}{HO\text{-}Y\text{-}X\text{-}D} \longrightarrow \underset{I}{A\text{-}CO\text{-}COO\text{-}Y\text{-}X\text{-}D}$$

Hal in der Formel IV steht dabei für ein Halogenatom wie Fluor, Chlor, Brom und Jod, vorzugsweise für Chlor oder Brom.

Die Umsetzung erfolgt im allgemeinen bei Temperaturen von -20 bis 100 °C, vorzugsweise bei 0 bis 80. °C.

Als Lösungsmittel und als Basen kommen in diesem Verfahren die vorstehend genannten in Betracht.

Verfahren C:

Außerdem erhält man die Verbindungen der Formel I dadurch, daß man einen Oxalylamidester der allgemeinen Formel VI in an sich bekannter Weise (Houben-Weyl, 4. Aufl. Bd. 8, S. 526ff) in einem inerten organischen Lösungsmittel mit einer Verbindung der allgemeinen Formel V umsetzt.

$$\underset{VI}{A\text{-}CO\text{-}COOCH_3} \quad + \quad \underset{V}{HO\text{-}Y\text{-}X\text{-}D} \longrightarrow \underset{I}{A\text{-}CO\text{-}COO\text{-}Y\text{-}X\text{-}D}$$

Die Umsetzung erfolgt im allgemeinen bei Temperaturen von -10 bis 150 °C, vorzugsweise bei 20 bis 100 °C.

Als Lösungsmittel eignen sich beispielsweise die vorstehend bei Verfahren A gennannten. Insbesondere kommen die genannten Alkohole in Betracht.

Als Basen kommen in diesem Verfahren neben den vorstehend genannten, insbesondere Natriumhydrid und Natriummethylat in Betracht.

Verfahren D:

Nach einem weiteren Verfahren erhält man die Verbindungen der Formel I auch dadurch, daß man das

Salz eines Oxalylamides der allgemeinen Formel VII in an sich bekannter Weise (Houben-Weyl, 4. Auf., Bd. 8, S. 542ff) in einem inerten organischen Lösungsmittel mit einer Verbindung der allgemeinen Formel VIII umsetzt.

$$\underset{VII}{A\text{-}CO\text{-}COO\text{-}Me} + \underset{VIII}{Z\text{-}Y\text{-}X\text{-}D} \longrightarrow \underset{I}{A\text{-}CO\text{-}COO\text{-}Y\text{-}X\text{-}D}$$

In der Formel VII steht Me für das Ion eines Alkalimetalls wie Lithium, Natrium, Kalium und Caesium.

Z in der Formel VIII steht für eine nukleophil verdrängbare Abgangsgruppe wie Halogen, biespielsweise Chlor, Brom oder Jod oder Alkyl- oder Arylsulfonyl wie insbesondere p-Tolylsulfonyl.

Die Umsetzung erfolgt im allgemeinen bei Temperaturen von 0 bis 150 °C, vorzugsweise bei 20 bis 120 °C.

Als Lösungsmittel eignen sich beispielsweise die vorstehend bei Verfahren A gennannten. Insbesondere kommen die folgenden in Betracht: N,N-Dimethylformamid, N,N-Dimethylacetamid und N-Methylpyrrolidon.

Als Basen kommen in diesem Verfahren im allgemeinen und im besonderen die vorstehend genannten in Betracht.

Die neuen Oxalsäurederivate der Formel I können chirale Zentren enthalten. Sie werden im allgemeinen als Racemate und gegebenenfalls als Diastereomerengemische erhalten. Einheitliche Diastereomere lassen sich bei einigen der neuen Verbindungen beispielsweise durch Säulenchromatographie oder aufgrund von Löslichkeitsunterschieden in reiner Form isolieren. Aus solchen gereinigten Diastereomeren kann man mit bekannten Methoden einheitliche Racemate und Enantiomere erhalten. Alle diese Verbindungen und Gemische werden von der vorliegenden Erfindung umfaßt. Für die Anwendung der neuen Azetidine als Wachstumsregulatoren sind sowohl die einheitlichen Diastereomeren bzw. Enantiomeren wie auch deren bei der Synthese angefallenen Gemische geeignet. Bevorzugt werden die letzteren verwendet.

Im Hinblick auf die bestimmungsgemäße Verwendung der Verbindungen I kommen als Substituenten folgende Reste in Betracht:

A ein über sein N-Atom gebundener Azetidinring, welcher ein oder zwei $C_1$-$C_3$-Alkylgruppen wie Methyl, Ethyl, Propyl und 1-Methylethyl tragen kann;

Y $C_2$-$C_{10}$-Alkylen wie Ethylen, Propylen, Butylen, Pentylen, Hexylen, Heptylen, Octylen, Nonylen oder Decylen, welches ein bis drei $C_1$-$C_3$-Alkylgruppen wie vorstehend bei A genannt, tragen kann;

X Sauerstoff oder Schwefel;

D $C_1$-$C_{20}$-Alkyl wie Methyl, Ethyl, geradkettiges oder verzweigtes Propyl, Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, vorzugsweise verzweigtes oder unverzweigtes $C_1$-$C_6$-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Di-methylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethyl-butyl, 2,3-Dimethylbutyl, 3,3-Di-methylbutyl, 1-Ethyl-butyl, 2-Ethylbutyl, 1,1,2-Trimethylpropyl, 1,2,2-Tri-methylpropyl, 1-Ethyl-1-methylpropyl und 1-Ethyl-2-methylpropyl;

insbesondere verzweigtes oder unverzweigtes $C_1$-$C_4$-Alkyl wie Methyl, Ethyl, Propyl, 1-Methylethyl, Butyl, 1-Methylpropyl, 2-Methylpropyl und 1,1-Dimethylethyl;

oder $C_3$-$C_{10}$-Alkenyl, insbesondere $C_3$-$C_6$-Alkenyl wie 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Di-methyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;

wobei diese Gruppen ein bis fünf Halogenatome wie Fluor, Chlor, Brom und Jod, vorzugsweise Fluor und/oder Chlor tragen können;

monocyclisches oder polycyclisches $C_3$-$C_{10}$-Cycloalkyl wie Cyclopropyl, Cyclobutyl, Cyclopentyl, Cyclohexyl, Cycloheptyl, Cyclooctyl, Menthyl, Norbornyl, Adamantyl und Tricyclodecanyl;

monocyclisches oder polycyclisches $C_3$-$C_{10}$-Cycloalkylmethyl wie Cyclopropylmethyl, Cyclobutylmethyl, Cyclopentylmethyl, Cyclohexylmethyl, Cycloheptylmethyl und Cyclooctylmethyl;

monocyclisches oder polycyclisches $C_5$-$C_{10}$-Cycloalkenyl wie Cyclopent-1-enyl, Cyclopent-2-enyl, Cyclopent-3-enyl, Cyclohex-1-enyl, Cyclohex-2-enyl, Cyclohex-3-enyl, Cyclohept-1-enyl, Cyclohept-2-enyl, Cyclohept-3-enyl, Cyclohept-4-enyl, Cyclooct-1-enyl, Cyclooct-2-enyl, Cyclooct-3-enyl und Cyclooct-4-enyl;

oder

monocyclisches oder polycyclisches $C_5$-$C_{10}$-Cycloalkenylmethyl wie Cyclopent-1-enylmethyl, Cyclopent-2-enylmethyl, Cyclopent-3-enylmethyl, Cyclohex-1-enylmethyl, Cyclohex-2-enylmethyl, Cyclohex-3-enylmethyl, Cyclohept-1-enylmethyl, Cyclohept-2-enylmethyl, Cyclohept-3-enylmethyl, Cyclohept-4-enylmethyl, Cyclooct-1-enylmethyl, Cyclooct-2-enylmethyl, Cyclooct-3-enylmethyl und Cyclooct-4-enylmethyl;

wobei diese gesättigten und einfach ungesättigten Ringe ein bis drei $C_1$-$C_4$-Alkylgruppen wie Methyl, Ethyl, Propyl, 1-Methylethyl, Butyl, 1-Methylpropyl, 2-Methylpropyl und 1,1-Dimethylethyl, vorzugsweise Methyl, 2-Methylpropyl und Dimethylethyl, insbesondere Methyl oder einen Phenylring tragen können;

Phenyl,

durch Phenyl substituiertes $C_1$-$C_6$-Alkyl wie vorstehend genannt, vorzugsweise $C_1$-$C_4$-Alkyl wie Methyl, Ethyl, Propyl, 1-Methylethyl, Butyl, 1-Methylpropyl, 2-Methylpropyl und 1,1-Dimethylethyl, insbesondere Methyl;

oder

durch Phenyl substituiertes $C_3$-$C_6$-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 3,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl;

wobei die aromatischen Reste ein bis fünf Halogenatome wie Fluor, Chlor, Brom und Jod, vorzugsweise Fluor und Chlor, und/oder ein bis drei der folgenden Gruppen tragen können: Nitro, $C_1$-$C_4$-Aklyl wie Methyl, Ethyl, Propyl, 1-Methylethyl, Butyl, 1-Methylpropyl, 2-Methylpropyl und 1,1-Dimethylethyl; $C_1$-$C_4$-Halogenalkyl wie insbesondere Trifluormethyl;

$C_1$-$C_4$-Alkoxy wie Methoxy, Ethoxy, Propyloxy, 1-Methylethoxy, Butyloxy, 1-Methylpropyloxy, 2-Methylpropyloxy und 1,1-Dimethylethoxy, $C_1$-$C_4$-Halogenalkoxy wie insbesondere Difluormethoxy und Triufluormethoxy;

$C_1$-$C_4$-Alkylthio wie Methylthio, Ethylthio, Propylthio, 1-Methylethylthio, Butylthio, 1-Methylpropylthio, 2-Methylpropylthio und 1,1-Dimethylethylthio.

Beispiele für insbesondere bevorzugte Oxalsäurederivate der allgemeinen Formel I sind in der folgenden Tabelle aufgeführt.

Tabelle

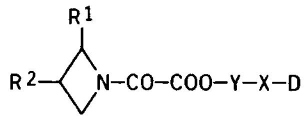

| D | Y | $R^1$ | $R^2$ | X |
|---|---|---|---|---|
| $CH_3$ | $CH_2$-$CH(CH_3)$ | H | H | O |
| $C_2H_5$ | $(CH_2)_2$ | H | H | O |
| $C_2H_5$ | $(CH_2)_2$ | $CH_3$ | H | O |
| $C_2H_5$ | $(CH_2)_2$ | H | H | S |
| $CH_3$ | $CH_2CH_2CH(CH_3)$ | H | H | O |
| n-$C_3H_7$ | $(CH_2)_2$ | H | H | O |
| i-$C_3H_7$ | $(CH_2)_2$ | H | H | O |
| $C_2H_5$ | $(CH_2)_3$ | H | H | O |
| n-$C_4H_9$ | $(CH_2)_2$ | $CH_3$ | $CH_3$ | O |
| n-$C_4H_9$ | $(CH_2)_3$ | H | H | O |
| n-$C_4H_9$ | $(CH_2)_2$ | H | H | S |
| n-$C_4H_9$ | $(CH_2)_4$ | H | H | O |
| n-$C_4H_9$ | $(CH_2)_6$ | H | H | O |
| n-$C_4H_9$ | $(CH_2)_8$ | H | H | O |
| sek.-$C_4H_9$ | $(CH_2)_4$ | H | H | O |
| sek.-$C_4H_9$ | $(CH_2)_4$ | $CH_3$ | H | O |
| sek.-$C_4H_9$ | $(CH_2)_{10}$ | H | H | O |
| i-$C_4H_9$ | $(CH_2)_4$ | H | H | O |
| i-$C_4H_9$ | $(CH_2)_5$ | H | H | O |
| n-$C_5H_{11}$ | $(CH_2)_2$ | H | H | O |
| n-$C_5H_{11}$ | $(CH_2)_3$ | H | H | O |
| n-$C_5H_{11}$ | $(CH_2)_8$ | H | H | O |
| $(CH_3)_3CCH_2$ | $(CH_2)_4$ | H | H | O |
| $(CH_3)_2CHCH_2CH_2$ | $(CH_2)_4$ | H | H | O |
| n-$C_6H_{13}$ | $(CH_2)_4$ | H | H | O |
| n-$C_6H_{13}$ | $(CH_2)_6$ | H | H | O |
| $(CH_3)_3CCH_2CH_2$ | $(CH_2)_4$ | H | H | O |
| n-$C_8H_{17}$ | $(CH_2)_4$ | H | H | O |
| 2-Ethylhexyl-1 | $(CH_2)_4$ | H | H | O |
| 2-Ethylhexyl-1 | $(CH_2)_6$ | H | H | O |
| 2-Ethylhexyl-1 | $(CH_2)_4$ | H | H | S |
| n-$C_{10}H_{21}$ | $(CH_4)_4$ | H | H | O |
| n-$C_{10}H_{21}$ | $(CH_2)_8$ | H | H | O |
| n-$C_{12}H_{25}$ | $(CH_2)_2$ | H | H | O |
| n-$C_{12}H_{25}$ | $(CH_2)_4$ | H | H | O |

Tabelle (Fortsetzung)

| D | Y | $R^1$ | $R^2$ | X |
|---|---|---|---|---|
| n-$C_{14}H_{29}$ | $(CH_2)_4$ | H | H | O |
| n-$C_{16}H_{33}$ | $(CH_2)_4$ | H | H | O |
| $CH_2$=$CHCH_2$ | $(CH_2)_2$ | H | H | O |
| $CH_2$=$CHCH_2$ | $(CH_2)_4$ | H | H | O |
| $CH_2$=$CHCH_2$ | $(CH_2)_4$ | $CH_3$ | H | O |
| $CH_2$=$CHCH_2$ | $(CH_2)_4$ | H | H | S |
| ClCH=$CHCH_2$ | $(CH_2)_4$ | H | H | O |
| $CH_2$=$CClCH_2$ | $(CH_2)_4$ | H | H | O |
| $CH_2$=$CBrCH_2$ | $(CH_2)_4$ | H | H | O |
| $CH_3CH$=$CHCH_2$ | $(CH_2)_4$ | H | H | O |
| Cl-$(CH_2)_3$ | $(CH_2)_4$ | H | H | O |
| Cl-$(CH_2)_4$ | $(CH_2)_4$ | H | H | O |
| Cl-$(CH_2)_6$ | $(CH_2)_4$ | H | H | O |
| Cl-$(CH_2)_8$ | $(CH_2)_4$ | H | H | O |
| Cyclopropyl | $(CH_2)_4$ | H | H | O |
| Cyclopropylmethyl | $(CH_2)_4$ | H | H | O |
| Cyclopropylmethyl | $(CH_2)_4$ | H | H | S |
| Cyclopentyl | $(CH_2)_4$ | H | H | O |
| Cyclopentyl | $(CH_2)_4$ | H | H | S |
| Cyclopentylmethyl | $(CH_2)_4$ | H | H | O |
| Cyclohexyl | $(CH_2)_2$ | H | H | O |
| Cyclohexyl | $(CH_2)_3$ | H | H | O |
| Cyclohexyl | $(CH_2)_4$ | H | H | O |
| Cyclohexyl | $(CH_2)_6$ | H | H | O |
| Cyclohexyl | $(CH_2)_8$ | H | H | O |
| Cyclohexyl | $(CH_2)_{10}$ | H | H | O |
| Cyclohexylmethyl | $(CH_2)_4$ | H | H | O |
| Cycloheptyl | $(CH_2)_4$ | H | H | O |
| 4-Methylcyclohexyl | $(CH_2)_4$ | H | H | O |
| 2-Methylcyclohexyl | $(CH_2)_4$ | H | H | O |
| 4-Isopropylcyclohexyl | $(CH_2)_4$ | H | H | O |
| 4-tert.-Butylcyclohexyl | $(CH_2)_4$ | H | H | O |
| 4-tert.-Butylcyclohexyl | $(CH_2)_6$ | H | H | O |
| 1-Decalyl | $(CH_2)_4$ | H | H | O |
| 2-Decalyl | $(CH_2)_4$ | H | H | O |
| 2-Norbornyl | $(CH_2)_4$ | H | H | O |
| 1,5-Dimethylbicyclo[2.3.1]octan-8-yl | $(CH_2)_4$ | H | H | O |
| Camphenyl | $(CH_2)_4$ | H | H | O |
| $C_6H_5$-$CH_2$ | $(CH_2)_2$ | H | H | O |

Tabelle (Fortsetzung)

| D | Y | $R^1$ | $R^2$ | X |
| --- | --- | --- | --- | --- |
| $C_6H_5-CH_2$ | $(CH_2)_3$ | H | H | O |
| $C_6H_5-CH_2$ | $(CH_3)_4$ | H | H | O |
| $C_6H_5-CH_2$ | $(CH_2)_6$ | H | H | O |
| $4-F-C_6H_4-CH_2$ | $(CH_2)_4$ | H | H | O |
| $4-Cl-C_6H_4-CH_2$ | $(CH_2)_4$ | H | H | O |
| $4-Br-C_6H_4-CH_2$ | $(CH_2)_4$ | H | H | O |
| $2,4-Cl_2C_6H_4-CH_2$ | $(CH_2)_4$ | H | H | O |
| $3,4-Cl_2C_6H_4-CH_2$ | $(CH_2)_4$ | H | H | O |
| $4-CH_3C_6H_4-CH_2$ | $(CH_2)_4$ | H | H | O |
| 4-tert.-$C_4H_9C_6H_4-CH_2$ | $(CH_2)_4$ | H | H | O |
| $4-F_3C\ C_6H_4-CH_2$ | $(CH_2)_4$ | H | H | O |
| $4-O_2N-C_6H_4-CH_2$ | $(CH_2)_4$ | H | H | O |
| $C_6H_5-CH_2-CH_2$ | $(CH_2)_4$ | H | H | O |
| $C_6H_5-CH_2CH_2$ | $(CH_2)_6$ | H | H | O |
| $C_6H_5-CH_2CH_2CH_2$ | $(CH_2)_4$ | H | H | O |
| $C_6H_5-CH=CHCH_2$ | $(CH_2)_4$ | H | H | O |
| $C_6H_5-(CH_2)_4$ | $(CH_2)_4$ | H | H | O |
| $C_6H_5-CH_2CH(CH_3)CH_2$ | $(CH_2)_4$ | H | H | O |
| 4-tert.-$C_4H_9-C_6H_4-CH_2CH(CH_3)CH_2$ | $(CH_2)_4$ | H | H | O |

Die erfindungsgemäßen wachstumsregulierenden Verbindungen I bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen

Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:

I. Man vermischt 90 Gewichtsteile der Verbindung Nr. 10 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 13 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 10 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 13 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 14 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 14 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 17 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 18 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

IX. Man vermischt 95 Gewichtsteile der Verbindung Nr. 19 mit 5 Gewichtsteilen N-Methyl-α-pyrrolidon und erhält eine Lösung, die zur Anwendung in Form kleinster Tropfen geeignet ist.

Die Applikation der wachstumsregulierenden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,01 bis 5 kg/ha, vorzugsweise 0,05 bis 1,0 kg/ha aktive Substanz (a.S.).

Die Verbindungen der Formel I können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren z.B. (Samenbeize, Bodenbehandlung, Blattapplikation oder Stamminjektion bei Bäumen)

d) von klimatischen Faktoren, z.B. Temperatur, Niederschlagsmenge, außerdem auch Tageslänge und Lichtintensität

e) von der Bodenbeschaffenheit (einschließlich Düngung),

f) von der Formulierung bzw. Anwendungsform des Wirkstoffs und schließlich

g) von den angewendeten Konzentrationen der aktiven Substanz.

Aus der Reihe der verschiedenartigen Anwendungsmöglichkeiten der erfindungsgemäßen Pflanzenwachstumsregulatoren der Formel I im Pflanzenanbau, in der Landwirtschaft und im Gartenbau, werden einige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist eine verminderte Intensität des Wachstums von Gräsern an Straßenrändern, Hecken, Kanalböschungen und auf Rasenflächen wie Park-, Sport- und Obstanlagen, Zierrasen und Flugplätzen, so daß der Arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Bei Obst- und anderen Bäumen lassen sich mit den Wachstumsregulatoren Schnittkosten einsparen. Außerdem kann die Alternanz von Obstbäumen durch Wachstumsregulatoren gebrochen werden.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.

B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren. Dabei können die Verbindungen der Formel I Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei

anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wie beispielsweise Baumwolle wesentlich.

D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite der Stomata reduziert wird
- eine dickere Epidermis und Cuticula ausgebildet werden
- die Durchwurzelung des Bodens verbessert wird und
- das Mikroklima im Pflanzenbestand durch einen kompakteren Wuchs günstig beeinflußt wird.

Die erfindungsgemäß zu verwendenden Wachstumsregulatoren der Formel I können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Infolge der hohen Pflanzenverträglichkeit kann die Aufwandmenge stark variiert werden.

Zur Verbreiterung des Wirkungsspektrums und zur Erzielung synergistischer Effekte können die erfindungsgemäßen Verbindungen I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die Verbindungen I allein oder in Kombination auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

## Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den nachstehenden Tabellen mit physikalischen Angaben aufgeführt.

### Beispiel 1

Azetidinoxamid-butoxyethylester

80 ml 2-Butoxyethanol und 17,2 g (0,12 Mol) Azetidinoxamid-methylester wurden mit 0,2 g Natriummethylat 3 Stunden bei 90 °C unter Stickstoffeinleitung gerührt. Nach Abkühlen auf 20 °C wurde das Gemisch mit 250 ml Ether versetzt, mit 50 ml Wasser gewaschen, über $Na_2SO_4$ getrocknet, im Vakuum eingeengt und anschließend fraktioniert destilliert.

Man erhielt 12,6 g (45,8 % der Theorie) Azetidinoxamid-butoxyethylester als farbloses Öl vom Sdp. 112-144 °C/0,3 mbar und $n_D^{22}$ = 1,4679.

### Beispiel 2

Azetidinoxamid-4-propoxybutylester

90 ml Dimethylformamid und 20 g (0,12 Mol) Azetidinoxamid-Kaliumsalz wurden mit 18 g (0,12 Mol) 4-Propoxybutylchlorid versetzt und drei Tage bei 60 °C nachgerührt. Nach Abkühlen wurde das Gemisch abgesaugt, im Vakuum eingeengt, der Rückstand in 200 ml Methylenchlorid aufgelöst, mit 50 ml Wasser gewaschen, über $Na_2SO_4$ getrocknet und eingeengt. Der Rückstand wurde nach 4 Stunden bei 90 °C und 0,2 mbar von den letzten Spuren Lösungsmittel befreit.

Man erhielt 17,8 g (61 % der Theorie) Azetidinoxamid-4-propoxybutylester als farbloses Öl, $n_D^{22}$ =

1,4572.

Tabelle 1

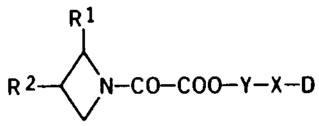

| Beispiel Nr. | D | Y | $R^1$ | $R^2$ | X | Sdp. (°C/mbar) Brechungsindex |
|---|---|---|---|---|---|---|
| 3 | $CH_3$ | $CH_2-CH(CH_3)$ | H | H | O | 125-130/0,3 |
| 4 | $C_2H_5$ | $(CH_2)_2$ | H | H | O | 129-133/0,4 |
| 5 | $CH_3$ | $CH_2CH_2CH(CH_3)$ | H | H | O | 137-139/0,4 |
| 6 | $n-C_3H_7$ | $(CH_2)_2$ | H | H | O | 141-143/0,5 |
| 7 | $i-C_3H_7$ | $(CH_2)_2$ | H | H | O | 146-150/0,5 |
| 8 | $C_2H_5$ | $(CH_2)_3$ | H | H | O | 137-139/0,2 |
| 9 | $n-C_4H_9$ | $(CH_2)_2$ | H | H | S | 180-184/0,3 |
| 10 | $n-C_4H_9$ | $(CH_2)_4$ | H | H | O | 156-159/0,3 |
| 11 | $n-C_4H_9$ | $(CH_2)_6$ | H | H | O | 163-166/0,3 |
| 12 | $n-C_4H_9$ | $(CH_2)_8$ | H | H | O | 190-193/0,3 |
| 13 | sek.-$C_4H_9$ | $(CH_2)_4$ | H | H | O | $n_D^{22}$ = 1,4670 |
| 14 | $i-C_4H_9$ | $(CH_2)_4$ | H | H | O | 152-153/0,3 |
| 15 | $i-C_4H_9$ | $(CH_2)_5$ | H | H | O | 161-163/0,4 |
| 16 | $(CH_3)_3C-CH_2$ | $(CH_2)_4$ | H | H | O | 170-172/0,4 |
| 17 | $CH_2=CH-CH_2$ | $(CH_2)_2$ | H | H | O | 131-133/0,3 |
| 18 | $CH_2=CH-CH_2$ | $(CH_2)_4$ | H | H | O | 137-139/0,3 |
| 19 | Cyclopentyl | $(CH_2)_4$ | H | H | O | 178-180/0,4 |
| 20 | Cyclohexyl | $(CH_2)_2$ | H | H | O | 171-173/0,4 |
| 21 | Cyclohexyl | $(CH_2)_4$ | H | H | O | 179-183/0,3 |

Anwendungsbeispiele

Die wachstumsregulierende Wirkung der Verbindungen der allgemeinen Formel I ließ sich durch folgende Versuche zeigen:

Die Wirkstoffe wurden

a) als 0,1 %-ige Lösung in Aceton oder

b) als 10 %-ige Emulsion in einem Gemisch aus 70 Gew.-% Cyclohexanol, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)

aufbereitet und entsprechend der gewünschten Konzentration mit Aceton im Fall von a) bzw. mit Wasser im Fall von b) verdünnt.

Als Kulturgefäße dienten Plastikblumentöpfe (Durchmesser ca. 12,5 cm; Volumen ca. 500 ml) mit lehmigem Sand mit etwa 3,0 % Humus als Substrat. Die Samen der Testpflanzen wurden nach Arten getrennt eingesät.

Bei Vorauflaufanwendung wurden die in Wasser suspendierten oder emulgierten Wirkstoffe direkt nach der Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Die Abdeckung bewirkt ein gleichmäßiges Keimen der Testpflanzen.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt.

Die Pflanzen wurden artenspezifisch bei Temperaturen von 10-25 °C bzw. 20-35 °C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Die beobachtete wachstumsregulierende Wirkung wurde bei Versuchsende durch die Messung der Wuchshöhe belegt. Die so gewonnenen Meßwerte wurden zur Wuchshöhe von nicht behandelten Pflanzen in Relation gesetzt. Als Vergleichsubstanz zur Beurteilung der wachstumsregulierenden Wirkung diente N-(2-Clorethyl)-N,N,N-trimethyl-ammonium chlorid (Beispiel A).

Die in den Gewächshausversuchen verwendeten Pflanzen waren: Sommerweizen (cv. "Ralle"), Sommergerste (cv. "Aramir"), Reis (cv. "Bahia"), Sonnenblumen (cv. "Spanners Allzweck") und Soja (cv. "Maple arrow").

Bei Nachauflaufanwendung von 1,5 mg/Gefäß zeigten die Verbindungen 1, 2, 3, 7, 8, 9, 13, 14 und 19 bei Sommerweizen (cv. "Ralle") und bei Sommergerste (cv. "Aramir") und die Verbindungen 1, 3, 7, 8 und 9 bei Reis (cv. "Bahia") eine bessere Wirkung als der bekannte Wachstumsregulator A.

Bei Nachauflaufanwendung von 6 mg/Gefäß zeigten die Verbindungen 2 und 8 bei Sonnenblumen (cv. "Spanners Allzweck") eine bessere Wirkung als der bekannte Wachstumsregulator A.

Bei Nachauflaufanwendung von 0,5 mg/Gefäß zeigten die Verbindungen 1, 2, 8 und 9 bei Soja (cv. "Maple arrow") eine bessere Wirkung als der bekannte Wachstumsregulator A.

## Patentansprüche

**1.** Oxalsäurederivate der allgemeinen Formel I,

A-CO-COO-Y-X-D I

in der die Substituenten folgende Bedeutung haben:

- A ein über sein N-Atom gebundener Azetidinring, welcher ein oder zwei $C_1$-$C_3$-Alkylgruppen tragen kann;
- Y $C_2$-$C_{10}$-Alkylen, welches ein bis drei $C_1$-$C_3$-Alkylgruppen tragen kann;
- X Sauerstoff oder Schwefel;
- D $C_1$-$C_{20}$-Alkyl oder $C_3$-$C_{10}$-Alkenyl, wobei diese Gruppen ein bis fünf Halogenatome tragen können;
  monocyclisches oder polycyclisches $C_3$-$C_{10}$-Cycloalkyl, $C_3$-$C_{10}$-Cycloalkylmethyl, $C_5$-$C_{10}$-Cycloalkenyl oder $C_5$-$C_{10}$-Cycloalkenylmethyl, wobei diese Ringe ein bis drei $C_1$-$C_4$-Alkylgruppen oder einen Phenylring tragen können;
  Phenyl, Phenyl-$C_1$-$C_6$-alkyl oder Phenyl-$C_3$-$C_6$-alkenyl, wobei die aromatischen Reste ein bis fünf Halogenatome und/oder ein bis drei der folgenden Gruppen tragen können: Nitro, $C_1$-$C_4$-Aklyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio.

**2.** Verfahren zur Herstellung der Verbindungen der Formel I gemäß Anspruch 1, dadurch gekennzeichnet, daß man ein Oxalylesterhalogenid der allgemeinen Formel II,

Hal-CO-COO-Y-X-D II

in der Hal für ein Halogenatom steht, in an sich bekannter Weise in einem inerten organischen Lösungsmittel in Gegenwart eine Base mit einem Azetidinderivat der allgemeinen Formel III,

A-H III

umsetzt.

**3.** Verfahren zur Herstellung der Verbindungen der Formel I gemäß Anspruch 1, dadurch gekennzeichnet, daß man ein Oxalylamidhalogenid der allgemeinen Formel IV,

A-CO-CO-Hal IV

in der Hal für ein Halogenatom steht, in an sich bekannter Weise in einem inerten organischen Lösungsmittel in Gegenwart eine Base mit einer Verbindung der allgemeinen Formel V,

HO-Y-X-D V

umsetzt.

4. Verfahren zur Herstellung der Verbindungen der Formel I gemäß Anspruch 1, dadurch gekennzeichnet, daß man einen Oxalylamidester der allgemeinen Formel VI,

$A\text{-}CO\text{-}COOCH_3$ VI

in an sich bekannter Weise in einem inerten organischen Lösungsmittel mit einer Verbindung der allgemeinen Formel V gemäß Anspruch 3 umsetzt.

5. Verfahren zur Herstellung der Verbindungen der Formel I gemäß Anspruch 1, dadurch gekennzeichnet, daß man das Salz eines Oxalylamides der allgemeinen Formel VII,

A-CO-COO-Me VII

in der Me für ein Erdaklalimetallion steht, in an sich bekannter Weise in einem inerten organischen Lösungsmittel mit einer Verbindung der allgemeinen Formel VIII,

Z-Y-X-D VIII

in der Z für eine nukleophil verdrängbare Abgangsgruppe steht, umsetzt.

6. Mittel zur Regulierung des Pflanzenwachstums, enthaltend eine regulativ wirksame Menge eines Azetidinderivats der Formel I gemäß Anspruch 1 und übliche inerte Zusatzstoffe.

7. Mittel zur Regulierung des Pflanzenwachstums, enthaltend 0,1 bis 95 Gew.% eines Azetidinderivats der Formel I gemäß Anspruch 1 und inerte Zusatzstoffe.

8. Verfahren zur Regulierung des Pflanzenwachstums, dadurch gekennzeichnet, daß man den Boden, die Pflanzen oder ihren Samen mit einem Azetidinderivat der Formel I gemäß Anspruch 1 behandelt.